# FASCICULE DE BREVET EUROPEEN

(11) **EP 1 041 977 B1**
(45) Date de publication et mention de la délivrance du brevet: **30.07.2008**
(21) Numéro de dépôt: 98965293.8
(22) Date de dépôt: 28.12.1998
(51) Int. Cl.: A61K 31/423, A61K 31/4439, A61P 17/00, A61P 17/02, A61P 17/06, A61P 17/10

(54) **UTILISATION D'ACTIVATEURS DE PPAR-GAMMA POUR TRAITER DES DESORDRES CUTANES**
VERWENDUNG VON PPAR-GAMMA AKTIVATOREN ZUR BEHANDLUNG VON HAUTKRANKHEITEN
USE OF PPAR-GAMMA ACTIVATORS FOR TREATING DERMATOLOGICAL DISORDERS

(30) Priorité: 31.12.1997 FR 9716808
(43) Date de publication de la demande: 11.10.2000
(73) Titulaire: GALDERMA RESEARCH & DEVELOPMENT, 06410 Biot (FR)
(72) Inventeur: RIVIER, Michel, F-06100 Nice (FR); SAFONOVA, Irina, F-06100 Nice (FR); MICHEL, Serge, F-06330 Roquefort les Pins (FR)
(74) Mandataire: Renard, Emmanuelle
(86) Numéro de dépôt international: PCT/FR1998/002894
(87) Numéro de publication internationale: WO 1999/034783

(56) Documents cités:
- EP-A- 0 347 056
- WO-A-90/14824
- WO-A-95/35108
- WO-A-96/09055
- WO-A-96/33724
- WO-A-98/08089
- WO-A-98/25598
- WO-A-98/32444
- WO-A-98/57631
- US-A- 5 443 844
- C.J. KAVANAUGH ET AL.: "CONJUGATED LINOLEIC ACID MODULATION OF PPAR-GAMMA IN MOUSE KERATINOCYTES" FASEB JOURNAL, vol. 12, no. 4, avril 1998, page A565 XP002078271
- S.A. KLIEWER ET AL.: "FATTY ACIDS AND EICOSANOIDS REGULATE GENE EXPRESSION THROUGH DIRECT INTERACTIONS WITH PEROXISOME PROLIFERATOR-ACTIVATED RECEPTORS ALPHA AND GAMMA" PROC. NATL. ACAD. SCI., vol. 94, no. 9, avril 1997, pages 4318-4323, XP002078272
- LEHMANN J M ET AL: "AN ANTIDIABETIC THIAZOLIDINEDIONE IS A HIGH AFFINITY LIGAND FOR PEROXISOME PROLIFERATOR-ACTIVATED RECEPTOR GAMMA (PPARGAMMA)" JOURNAL OF BIOLOGICAL CHEMISTRY, vol. 270, no. 22, 2 juin 1995, pages 12953-12956, XP000577082
- M.D. BREGMAN ET AL.: "INHIBITION OF HUMAN MELANOMA GROWTH BY PROSTAGLANDIN A,D AND J ANALOGUES" CANCER RESEARCH, vol. 46, no. 6, 1986, pages 2740-2744, XP002078273
- D.R. BUCKLE ET AL.: "NON THIAZOLIDINEDIONE ANTIHYPERGLYCAEMIC AGENTS.1" BIOORGANIC & MEDICINAL CHEMISTRY LETTERS, vol. 6, no. 17, 1996, pages 2121-2126, XP002078274

## Description

La présente invention concerne l'utilisation d'au moins un activateur des récepteurs de type PPAR-γ choisi parmis les composés indiqués dans les revendications 1 et 8 dans la préparation d'une composition pharmaceutique, la composition étant destinée à traiter les désordres cutanés liés à une anomalie de la différenciation des cellules épidermiques choisis parmi les maladies listées dans les revendications 1 et 8.

Il existe de nombreux désordres cutanés liés à une anomalie de la différenciation des cellules épidermiques, on peut citer, à titre d'exemple, le psoriasis, l'eczéma, les dermatites, l'acné vulgaire, les kératoses, dont l'ichtyose, et les cancers cutanés. Cette anomalie de la différenciation des cellules épidermiques est généralement accompagnée d'une hyperprolifération des cellules épidermiques. Pour traiter ces désordres, différentes approches pharmaceutiques ont été envisagées. Mais, aucun traitement aujourd'hui ne donne une entière satisfaction. Ainsi, il existe un besoin d'améliorer les traitements existants.

Les peroxisomes sont des petits organites proches des mitochondries contenant une série d'enzymes propres au métabolisme de l'eau oxygéné (catalase, urate-oxydase, D-aminoacide-oxydase) et des enzymes de la β-oxydation des acides gras. Les proliférateurs des peroxisomes sont principalement des groupes de produits chimiques qui comprennent les hypolipidémiants, tel que le clofibrate, les herbicides et les plastiques industriels, tels que les esters de phtalate. Ces proliférateurs des peroxisomes sont des carcinogènes non génotoxiques qui activent des récepteurs, appelés PPARs, faisant partie de la super-famille des récepteurs nucléaires stéroïdiens. Ces récepteurs peuvent être activés par les proliférateurs de peroxisome, ils peuvent être également activés par des acides gras naturels, ils stimulent ainsi l'expression de gènes codant pour des enzymes impliqués dans la β-oxydation peroxisomale et mitochondriale ou encore pour la P450-4A6 β-hydroxylase d'acide gras.
L'ensemble des références suggère un rôle des PPARs dans la régulation du métabolisme et l'homéostasie des lipides.
Les récepteurs PPARs activent la transcription en se liant à des éléments de séquence d'ADN, appelés les éléments de réponse des proliférateurs de peroxisome (PPRE), sous forme d'un hétérodimère avec les récepteurs X des rétinoïdes (appelés les RXRs).
Trois sous-types de PPARs humains ont été identifiés et décrits : les PPARα, PPARγ et PPARδ (ou NUC1).

Il a été décrit dans la demande de brevet WO 96/33724 que des composés sélectifs des PPARγ, tels qu'une prostaglandine-J2 ou -D2 sont des actifs potentiels pour le traitement de l'obésité et du diabète.
Buckle et al décrit l'activité antihyperglycémiante de dérivés benzooxazoles y compris l'acide 3-{4-[2-(Benzooxazol-2-yl-méthyl-amino)-éthoxy]-phényl}-2-éthoxy-propionique. En outre, ce document précise leur similarité structurale avec les agonistes PPAR-gamma tels que la ciglitazone et la 5-{4-[2-(méthyl-pyridin-2-yl-amino)-éthoxy]-benzyl}-thiazolidine-2,4-dione, et conclut que les dérivés benzooxazoles agissent au moins partiellement en tant qu'agonistes PPAR-gamma ("Non thiazolidinedione antihyperglycaemic agents. 1: α-heteroatom substituted β-phenylpropanoic acids" Bioorganic & Medicinal Chemistry Letters, vol.6, no.17, 1996, pages 2121-2126).
Certaines thiazolidinediones y compris la 5-{4-[2-(méthyl-pyridin-2-yl-amino)-éthoxy]-benzyl}-thiazolidine-2,4-dione ont été décrites comme agonistes spécifiques des récepteurs PPARγ (Lehmann et al, « An antidiabetic thiazolidinedione is a high affinity ligand for peroxisome proliferator-activated receptor γ », Journal of Biological Chemistry, Vol.270, no.22, 2 juin 1995, pages 12953-12956).
Par ailleurs, il a été décrit dans la demande de brevet WO 95/35108 que des thiazolidinediones, plus particulièrement le ciglitazone, présentent une activité dans le traitement du psoriasis en inhibant la prolifération des kératinocytes.
Enfin, WO 98/08089 décrit un modèle in vitro de glande sébacée ; ce modèle permet d'identifier des composés destinés à traiter des désordres liés à la formation du sébum. Ce document mentionne également des compositions topiques à base de stimulateurs non androgènes tels que notamment l'agoniste PPAR-gamma 5- {4- [2- (méthyl- pyridin-2-yl-amino) -éthoxy]-benzyl}-thiazolidine-2,4-dione pour traiter des désordres résultant d'une production insuffisante de sébum tels que l'eczéma, les ichtyoses et les lichens.

La Demanderesse vient de découvrir que, lorsque la différenciation de kératinocytes humains est induite avec une concentration élevée en calcium, le taux d'expression des PPARs, plus particulièrement de PPARγ, est augmenté. Aussi, elle a constaté que le taux d'expression des PPARs, plus particulièrement de PPARγ, dans des peaux lésées psoriasiques est notablement réduit par rapport à celui dans des peaux non lésées.

Ainsi, la présente invention a pour objet l'utilisation d'au moins un activateur des récepteurs de type PPAR-γ choisi parmi les composés indiqués dans les revendications 1 et 8 dans la préparation d'une composition pharmaceutique, la composition étant destinée à traiter les désordres cutanés liés à une anomalie de la différenciation des cellules épidermiques choisis parmi les maladies listées dans les revendications 1 et 8.

La composition pharmaceutique est, de préférence, une composition dermatologique.

Par activateur des récepteurs de type PPAR-γ, on entend selon l'invention tout composé qui présente dans un test de transactivation, tel que décrit dans Kliewer et al., Nature 358, 771-774, 1992, une AC50 relative au PPAR-γ inférieure ou égale à 1 µM.

De préférence, l'activateur des récepteurs de type PPAR-γ présente une AC50 relative au PPAR-γ inférieure ou égale à 200 nM et avantageusement inférieure ou égale à 50 nM.

De préférence, l'activateur des récepteurs de type PPAR-γ est spécifique, c'est à dire qu'il présente un rapport R1 d'AC50 relative au PPAR-γ sur l'AC50 relative au PPARα inférieur ou égal à 10⁻¹. De préférence, R1 est inférieur ou égale à 0,05, et plus avantageusement inférieur ou égale à 0,02.

Une AC50 est la concentration en composé "activateur" nécessaire pour présenter 50% d'une activité enzymatique (luciférase) rapporteuse de l'activation due au composé via un des récepteurs PPARs, et plus particulièrement de type PPAR-α ou PPAR-γ.

Les composés utilisés dans la présente invention sont :
5-{4-[2-(méthyl-pyridin-2-yl-amino)-éthoxy]-benzyl}-thiazolidine-2,4-dione
acide-3-{4-[2-(Benzooxazol-2-yl-méthyl-amino)-éthoxy]-phényl}-2-éthoxy-propionique ;
acide(+)-3-{4-[2-(Benzooxazol-2-yl-méthyl-amino)-éthoxy]-phényl}-2-éthoxy-propionique ;
acide(-)-3-{4-[2-(Benzooxazol-2-yl-méthyl-amino)-éthoxy]-phényl}-2-éthoxy-propionique.

Il est d'autant plus surprenant que les composés utilisés dans la présente invention traitent ce type de désordres cutanées, dans la mesure où certains d'entre eux présentent une faible, voire ne présentent aucune, activité inhibitrice de la prolifération des kératinocytes, tels que la 5-{4-[2-(methyl-pyridin-2-yl-amino)-éthoxy]-benzyl}-thiazolidine-2,4-dione.

De préférence, l'activateur des récepteurs de type PPAR-γ utilisé présente un % d'inhibition de prolifération des kératinocytes inférieur ou égal à 20% lorsqu'il est utilisé à une concentration inférieure ou égale à 100nM (voir exemple ci-dessous).

La composition pharmaceutique selon l'invention comprend un milieu physiologiquement acceptable.

D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

Parmi les désordres liés à une anomalie de la différenciation des cellules épidermiques, plus particulièrement les kératinocytes, on peut citer plus particulièrement le psoriasis, l'eczéma, le lichen plan, les lésions de la peau associées à un lupus, les dermatites, telles que les dermatites atopique, sébhorréïque ou solaire, les kératoses, telles que la kératose sébhorréïque, sénile, actinique, photo-induite ou folliculaire, l'acné vulgaire, les kéloïdes, les nevi, les verrues, les ichtyoses.

Parmi les désordres liés à une anomalie de la différenciation des cellules épidermiques, on cite de préférence, les anomalies de la fonction barrière, telles que la dermatite atopique, l'eczéma et le psoriasis.

L'administration de la composition selon l'invention peut être effectuée par voie entérale, parentérale ou topique. De préférence, la composition pharmaceutique est conditionnée sous une forme convenant à une application par voie topique.

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les composés sont utilisés selon l'invention sont généralement administrés à une dose journalière d'environ 0,001 mg/kg à 100 mg/kg en poids corporel en 1 à 3 prises.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter soit sous forme anhydre, soit sous forme aqueuse.

Les composés sont utilisés par voie topique à une concentration généralement comprise entre 0,001 % et 10 % en poids, de préférence entre 0,01 et 1 % en poids, par rapport au poids total de la composition.

Les compositions telles que décrites précédemment peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; les acides eicosa-5,8,11,14-tétraynoïque et eicosa-5,8,11-triynoïque, leurs esters et amides et enfin les rétinoïdes.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre nullement limitatif, plusieurs exemples destinés à illustrer la présente invention.

### EXEMPLE 1

### AC50 et R1 des produits activateurs de PPARγ tels que décrits ci-dessus.

La méthode utilisée pour déterminer les AC50 est celle décrite décrit dans Kliewer et al., Nature 358, 771-774, 1992. Ainsi, le pouvoir activateur via PPARγ ou PPARα de molécules peut être évalué avec un test de transactivation dans lesquels les cellules HeLa ont été cotransfectées avec un vecteur d'expression codant pour ces récepteurs et un plasmide rapporteur contenant un élément de réponse PPRE cloné en amont d'une partie du promoteur du virus SV40 et du gène luciférase. Les cellules cotransfectées sont traitées pendant 24 heures avec les molécules à tester et l'activité de la luciférase est déterminée par luminescence.

Le tableau 1 rassemble les résultats. Pour chaque molécule, les résultats sont exprimés en nM par la valeur d'AC50 qui représente donc la concentration de molécule à tester donnant 50% de l'activité maximale.

**Tableau 1**

| | AC50 (nM) - PPARα | AC50 (nM) - PPARγ | R1 |
|---|---|---|---|
| Composé A | 2800 | 70 | 0.025 |
| Composé B | 2900 | 37 | 0,013 |
| Composé C | 4600 | 130 | 0,028 |
| Composé D | 1450 | 5 | 0,003 |

| | | | |
|---|---|---|---|
| Composé A : 5-{4-[2-(méthyl-pyridin-2-yl-amino)-éthoxy]-benzyl}-thiazolidine-2,4-dione ; Composé B : acide-3-{4-[2-(Benzooxazol-2-yl-méthyl-amino)-éthoxy]-phényl}-2-éthoxy-propionique ; Composé C : acide (+)-3-{4-[2-(Benzooxazol-2-yl-méthyl-amino)-éthoxy]-phényl}-2-éthoxy-propionique ; Composé D : acide(-)-3-{4-[2-(Benzooxazol-2-yl-méthyl-amino)-éthoxy]-phényl}-2-éthoxy-propionique. | | | |

### EXEMPLE 2

### Détermination de la prolifération des kératinocytes

Les kératinocytes utilisés ont été obtenus à partir de spécimen de peau provenant de chirurgie plastique. Ils sont utilisés a deuxième passage.

Les kératinocytes sont cultivées à 37°C dans une atmosphère humide (5% de CO2) selon la méthode de Rheinwald et Green en présence de fibroblastes 3T3 traités par la mitocyne dans du milieu MEM contenant 10% de sérum de veau foetal (SVF), 0,4 µg/ml d'hydrocortisone, 10ng/ml d'EGF et 10⁻⁹ M de cholératoxine. Les cellules 3T3 sont ensemencées 24 heures avant les kératinocytes à raison de 15000 cellules par cm² dans des puits de cluster de 10cm². Ensuite, les kératinocytes sont ensemencés à raison de 4000 cellules par cm².

Les cellules sont traitées 4 heures parès l'ensement des kératinocytes avec les produits dilués dans le DMSO. La concentration finale de DMSO dans le milieu de culture n'excède pas 0,2 % (v/v). Après trois jours de culture, la solution de 5-bromo-2'-déoxyuridine (BrdU, cell proliferation kit de Boehringer ref 1 674629) diluée au 1/100 est ajoutée au milieu de culture.

Après dénaturation de l'ADN, l'anticorps anti BrdU-POD (peroxydase) est ajouté selon les indications du fabriquant. Après 1 heure d'incubation, la solution de substrat est ajoutée et la lecture de la densité optique est effectuée à 370nM dans un lecteur ELISA.

Ainsi, le composé A décrit dans l'exemple 1 présente un % d'inhibition de prolifération des kératinocytes inférieur ou égal à 20% lorsqu'il est utilisé à une concentration inférieure ou égale à 100nM.

### EXEMPLE 3

### MATERIAUX ET METHODES

### Conditions de culture des cellules

Les kératinocytes humains normaux (KHN) ont été isolés de la peau humaine obtenue après une chirurgie plastique et cultivée par la méthode de Rheinwald and Green (Rheinwald J.G. and Green, H. Serial cultivation of strains of humain epidermal keratinocytes : the formation of keratinizing colonies from single cells. Cell, 1975, 6, 331-343). Les cellules ont été cultivées dans un milieu complet (MCDB 153) contenant 0,5mM de CaCl₂. Le milieu complet MCDB 153 est composé d'un milieu basal (KBM, Clonetics, San Diego, CA) auquel on a rajouté 0,4 % (v/v) d'extrait pituitaire bovin, 5 µg/ml d'insuline et 10ng/ml de facteur de croissance épidermique (EGF). Quand les cellules ont atteint environ 60 % de confluence (jour 0), le milieu est remplacé par un milieu complet MCDB 153 contenant soit 0,15mM de CaCl₂ (milieu bas calcium), soit 1,15mM de CaCl₂ (milieu haut calcium). Le milieu est changé tous les deux jours.

### Epiderme reconstruit sur un équivalent dermal de collagène de type I

Les cellules de l'épiderme interfolliculaire adulte, isolées de la peau de sein humain obtenue après une chirurgie plastique, ont été amplifiées (Rheinwald J.G. and Green, H. Serial cultivation of strains of humain epidermal keratinocytes : the formation of keratinizing colonies from single cells. Cell, 1975, 6, 331-343) et stockées dans de l'azote liquide. Les cellules ont été décongelées et ensemencées sur un équivalent de derme de collagène de type | (Asselineau, D., Bernard, B.D. and Darmon, M. Three-dimensional culture of human keratinocytes on a dermal equivalent. A model to study epidermal morphogenesis and differentiation in vitro. In: Maibach, H., Lowe, N. (eds.) Models in Dermatology. Karger, Basel, 1987, 1987, 1-7). Les cultures ont été dans un premier temps gardées submergées dans le milieu de culture pendant une semaine pour obtenir une monocouche confluente (jour 0) et ont ensuite été mises à l'interface air-liquide pour produire un epithelium stratifié et kératinisé. Le milieu de culture a consisté en un milieu essentiel minimal (MEM) auquel on a ajouté 10 % (v/v) de serum de veau fétal, EGF (10 ng/ml), hydrocortisone (0,4 µg/ml) et une toxine de choléra (10-9M). Le milieu a été changé trois fois par semaine.

La morphologie de la peau reconstruite a été évaluée en colorant des coupes verticales paraffinées avec de l'hemalum-phloxin-saffron.

### Biopsies

Des biopsies ont été obtenues à partir de peau psoriatique impliquée et non-impliquée après accord des patients. Les biopsies ont été immédiatement immergées dans une solution d'extraction d'ARN (4M guanidium thiocyanate). Elle ont été ensuite congelées dans de l'azote liquide et gardées à -80°C jusqu'à leur utilisation.

### Isolement des ARN

Tous les ARN provenant des kératinocytes en culture ou de peau reconstruite ont été isolés en utilisant la méthode Trizol (Gibco BRL), selon la procédure du fabricant et conservés à -80°C jusqu'à leur utilisation. Tous les ARN provenant des biopsies de peau ont été préparés tels que décrits par Chomczynski and Saachi (Chomczynski, P. and Sacchi, N. Single-step method of RNA isolation by acid guanidinium thiocyanate-phenol-chloroform extration. Anal.Biochem., 1987, 162, 156-159).

### RT-PCR et PCR semi-quantitative

Les oligonucléotides de PCR ont été synthétisés par Gibco BRL (France) et ont la séquence décrite ci-dessous :
oligonucléotide sens GAPDH (5'-AATCCCATCACCATCTTCCA-3') et oligonucléotide antisens (5'-GTCATCATATTTGGCAGGTT-3') ; oligonucléotide sens pour le PPARα (5'-TCATCAAGAAGACGGAGTCG-3') et oligonucléotide antisens (5'-CGGTTACCTACAGCTCAGAC-3') ; oligonucléotide sens pour le PPARγ (5'-ATGACAGCGAACTTGGCAATA-3') et oligonucléotide antisens (5-CGAACTGGAAGAAGGGAAAT-3') ; oligonucléotide sens pour la kératine 1 (5-AGTTCCAGCGTGAGGTTTGT-3') et oligonucléotide antisens (5'-GGGACTGAGATTGCCACTGA-3') ; oligonucléotide sens pour la loricrine (5'-ACCACGGAGGCCGAAGGAGTT-3') oligonucléotide antisens (5'-CTGGGGTTGGGAGGAGGTAGTTG-3') ; oligonucléotide sens pour la transglutaminase type | (TGI) (5'-GCGGCAGGAGTATGTTCTTA-3') et oligonucléotide antisens (5'-AGGGATGTGTCTGTGTCGTG-3'). Les produits amplifiés sont égaux à 558 bp pour GAPDH ; 211, 287 et 341 bp pour le PPARα, δ et γ, respectivement ; 250 bp pour la kératine 1 ; 189 bp pour la loricrine et 444 bp pour la TGI.

La RT-PCR a été réalisée en utilisant 5µg des ARN extraits des cellules cultivées, des peaux reconstruites ou des biopsies de la peau. Après dénaturation dans de l'eau traitée au diéthylpyrocarbonate pendant 10 minutes à 70°C, les ARN ont été reverse-transcrits en ADNc en utilisant le kit "SuperScript Il RNase H- reverse transcriptase (10 unités/réaction, Gibco BRL) et 0,5µg d'oligo(dT) comme primer, à 42°C pendant 50 min, pour un volume de tampon total de 20µl (20mM Tris-HCl, pH 8,4 - 50mM KCl, 1,5mM MgCl2, 1mM dNTP, 10mM DTT, 20 unités de l'inhibiteur de RNase). Le mélange a été inactivé à 70°C pendant 15 min et traité par RNaseH à 37°C pendant 20 min. Dans chaque cas, des échantillons ne contenant aucune transcriptase reverse (contrôle négatif) ont été inclus. L'amplification par PCR a été réalisée avec un appareil de PTC 225 (MJ Research), après une période d'une minute de dénaturation à 94°C, sous les conditions suivantes : dénaturation à 94°C pendant 30 secondes, appariement à 55°C pendant 30 secondes, et extension à 72°C pendant 30 secondes, sur un total de 30 cycles.

Le mélange réactionnel contient 20 mM de Tris-HCl, pH 8,4, 50 mM de KCl, 1,5 mM de MgCl₂, 0,1 µM d'oligonucléotide primers, 100 µM de dNTP, 0,5 unités de Polymerase DNA Taq et 5 µl du mélange d'ADNc dilué à 1/100. Le produit final a été chauffé pendant 3 minutes à 72°C. Dans chaque cas, des contrôles positifs de RT (contenant de l'ADNc codant pour les PPARs) et un contrôle négatif de PCR (sans ADN) ont été inclus. Les produits de PCR ont été séparés par électrophorèse sur gels d'agorose à 2 % contenant 1 µg/ml de bromure d'ethidium, photographiés et leur identité a été confirmée par Southern-blot.

La PCR semi-quantitative a été réalisée comme décrit ci-dessus, excepté que les concentrations de dNTP ont été de 100µM de dATP, dGTP,dTTP, 10 µM de dCTP, 0,5 µCi de [32P] dCTP. Les produits de PCR ont ensuite été séparés avec des gels d'acrylamide à 6 % (poids/v). La radioactivité de chaque bande a été quantifiée par la technique du phosphorimaging. Les écrans ont été criblés (scanned) en utilisant un FUJI BAS 2000 et le signal a été quantifié en unités LSP (Photo Stimulating Luminescence) en utilisant un logiciel d'analyse d'image (Tina). Les résultats ont été analysés pour chaque échantillon en comparant l'intensité de la bande avec celle de GAPDH. Un nombre optimal de cycles de PCR a été déterminé dans la région d'amplification linéaire. Le mélange d'ADNc a été dilué de 10 en 10 pour contrôler la corrélation linéaire entre l'intensité du signal radioactif et la quantité initiale d'ADN.

### RESULTATS

Les premières expériences, utilisant l'analyse par Northern-blot d'ARN totaux provenant de KHN en culture, ont montré que PPARδ est présent en quantités presque égales dans les kératinocytes non-différenciés et différenciés, alors que les PPARα et -γ n'ont pas été détectés.
Pour analyser l'expression des PPARs, l'analyse par RT-PCR a été réalisée en utilisant des oligonucléotides spécifiques des sous-types de PPAR. Les produits de PCR de 211 bp, 287 bp et 341 bp correspondant respectivement au PPARα, -δ et -γ ont été obtenus après electophorèse. La spécificité des produits de PCR a été vérifiée en utilisant des plasmides contenant des ADNc codant pour les PPARs. De plus, l'analyse par Southern-blot a été effectuée pour identifier les produits de PCR. Les trois sous-types de PPAR ont été détectés à la fois dans les KHN non-différenciés obtenus à 60 % de confluence dans un milieu bas calcium et dans les KHN différenciés obtenus après 4 jours de culture dans un milieu haut calcium.

Ensuite, l'analyse semi-quantitative par PCR a été réalisée pour comparer le niveau d'expression des sous-types de PPARs dans les KHN cultivés dans un milieu de bas calcium ou dans un milieu de haut calcium. L'expression du gêne de PPARα s'accroît lentement dans un milieu de bas calcium du jour 0 au jour 4 (1,7 fois) et dans un milieu de haut calcium (2,6 fois). Après 4 jours, l'expression du gêne de PPARδ a augmenté aussi bien dans les milieux de bas calcium que de haut calcium (4,5 et 5,8 fois, respectivement). Le niveau d'expression du gêne de PPARγ reste inchangé pendant 4 jours dans un milieu de bas calcium, tandis qu'il augmente de manière surprenante entre le 3^{ème} et le 4^{ème} jour après que les cellules aient été cultivées dans un milieu de haut calcium (4,5 fois).

Puisque la différenciation des kératinocytes est incomplète dans des conditions de culture immergées, nous avons utilisé la peau reconstruite *in vitro* qui montre une stratification et une kératinisation de l'épiderme plus complète, pour étudier l'expression de PPARs. 2 jours après l'émersion, uniquement deux ou trois couches de kératinocytes sont présentes, 2 jours plus tard, les kératinocytes sont organisés dans un mince epithelium multicouches, tandis qu'au bout de 7 jours, l'épiderme est composé d'un epithelium qui ressemble à l'épiderme humain normal. Des études par immunofluorescence ont montré que les marqueurs de différenciation, tels que l'involucrine, la filagrine et TGI (transglutaminase I), sont exprimés et localisés tels que décrits dans Asselineau, D., Bernard, B.D. and Darmon, M. Three-dimensional culture of human keratinocytes on a dermal équivalent. A model to study epidermal morphogenesis and differentiation in vitro. In: Maibach, H., Lowe, N. (eds.) Models in Dermatology. Karger, Basel, 1987, 1-7.

Le niveau d'expression des trois sous-types de PPAR pendant la reconstruction de l'épiderme est le suivant :
L'ARN de PPARα est légèrement augmenté (2 fois) après 7 jours d'émersion. Le niveau de PPARδ reste constant du jour 0 au jour 7. Par contre, l'expression de PPARγ augmente de 7 fois entre le jour 0 et le jour 7.

Le mode d'expression des sous-types de PPAR durant la reconstruction de la peau a été comparé à celui des marqueurs de différenciation du kératinocyte. L'expression maximale de la kératine 1, de la loricrine et de la TGI a été observée 7 jours après l'émersion.

Pour analyser l'expression des sous-types de PPARs dans la peau humaine, les ARN totaux ont été préparés à partir d'épiderme psoriatique non-lésé ou lésé et soumis à l'analyse semi-quantitative de RT-PCR. L'expression de PPARα est simplement légèrement diminuée dans la peau lésée alors que l'expression de PPARγ est diminuée considérablement (environ 3,5 fois). Cependant, l'expression de PPARδ augmente dans l'épiderme lésé par rapport à l'épiderme non lésé.

### DISCUSSION

Les trois sous-types de PPAR ont été décrits dans les amphibiens, les rongeurs et les humains. (Issemann, I. and Green, S. Activation of a member of the steroid hormone receptor superfamily by peroxisome proliferators. 1990, Nature, 347, 647-650 - Sher, T., Yi, H.-F., McBride, O.W. and Gonzalez, F.J. cDNA cloning, chromosomal mapping, and functional characterization of the human peroxisome proliferator activated receptor. Biochemistry, 1993, 32, 5598-5604 - Zhu, Y., Alvares, K., Huang, Q., Rao, H.S. and Reddy, J.K. Cloning of a new member of the peroxisome proliferator-activated receptor gene family from mouse liver. J. Biol. Chem., 1993, 268, 26817-26280 - Greene, M.E., Blumberg, B. McBride, O.W., Yi, H.F., Kronquist, K., Kwan, K., Hsieh, L., Greene, G. and Nimer, S.D. Isolation of the human peroxisome proliferator activated receptor gamma cDNA : expression in hematopoietic cells and chromosomal mapping. Gene Expression, 1995, 4, 281-299 - Elbrecht, A., Chen, Y., Cullinan, C.A., Hayes, N., Leibowitz, M.D., Moller, D.E. and Berger, J. Molecular cloning, expression and characterization of human 1 and γ2. Biochem. Biophys. Res. Comm., 1996, 224, 431-437 - Lambe, K.G. and Tugwood, J.D. A human peroxisome-proliferator-activated receptor-γ is activated by inducers of adipogenesis, including thiasolidinedione drugs. Eur.J. Biochem., 1996, 239, 1-7 - Schmidt, A., Endo, N., Rutledge, S.J., Vogel, R., Shinar, D. and Rodan, G.A. Identification of a new member of a steroid hormone receptor superfamily that is activated by a peroxisome proliferator and fatty acids. Mol.Endocrinol., 1992, 6, 1634-1641 - Kliewer, S.A., Forman, B.M., Blumberg, B., Ong, E.S., Borgmeyer, U., Mangelsdorf, D.J., Umesoto, K. and Evans, R.M. Differential expression and activation of a family of murine peroxisome proliferator-activated receptor. Proc. Natl. Acad. Sci. USA, 1994, 91, 7355-7359 - Amri, E.-Z, Bonino F., Ailhaud, G., Abumrad, N.A. and Grimaldi, P.A. Cloning of a protein that mediated transcriptional effects of fatty acids in preadipocytes. J. Biol. Chem., 1995, 270, 2367-2371 - Dreyer, C., Krey, G., Keller, H., Givel, F., Helftenbein, G. and Wahli, W. Control of the peroxisomal beta-oxydation pathway by a novel family of nuclear hormone receptors. Cell, 1992, 68, 879-887). PPARα est fortement exprimé dans des tissus montrant un niveau élevé d'oxydation des acides gras et de métabolisme peroxisomal tel que le foie, le coeur, le rein et l'intestin. PPARδ est exprimé abondamment et de manière omniprésente, tandis que l'expression de PPARγ se trouve de manière prédominante dans des tissus présentant une lipogénèse active tel que le tissu adipeux blanc mais il est également trouvé dans les cellules du système immunitaire (Issemann, I. and Green, S. Activation of a member of the steroid hormone receptor superfamily by peroxisome proliferators. 1990, Nature, 347, 647-650 - Kliewer, S.A., Forman, B.M., Blumberg, B., Ong, E.S., Borgmeyer, U., Mangelsdorf, D.J., Umesoto, K. and Evans, R.M. Differential expression and activation of a family of murine peroxisome proliferator-activated receptor. Proc. Natl. Acad. Sci. USA, 1994, 91, 7355-7359 -Lehmann, J.M., Moore, L.B., Smih-Oliver, T.A., Wilkinson, W.O., Wilson, T.M. and Kliewer, S.A. An antidiabetic thiasolidinedione is a high affinity ligand for peroxisome proliferator activated receptors γ (PPARγ) J. Biol. Chem., 1995, 270, 12953-12956 - Braissant, O., Foufelle, F., Scotto, C., Dauca, M. and Wahli, W. Differential expression of proxisome proliferator-activated receptor (PPARs) : tissue distribution of PPAR-α, -β, and -γ in the adult rat, Endocrinology, 1996, 137, 354-366 - Tontonoz, P., Hu, E., Graves, R.A., Budavari, A.I. and Spiegelman, B.M. mPPARγ2n tissue-specific regulator of an adipocyte enhancer. Genes Dev., 1994, 8, 1224-1234). La distribution spécifique tissulaire des trois sous-types de PPAR pourrait expliquer leur action physiologique différente. Le PPARα et le PPARγ semblent réguler les deux branches de l'homéostase lipidique, c'est à dire respectivement le catabolisme des acides gras et la lipogenèse. Tandis que l'expression omniprésente de PPARδ suggère une fonction biologique générale mais encore inconnue.

Le processus de différenciation des kératinocytes de l'épiderme est accompagné d'une accumulation de lipides spécifiques responsables de la formation de la fonction barrière de l'épiderme. Cependant, nous connaissons peu de choses sur le niveau d'expression et le rôle des sous-types de PPAR dans l'épiderme. Une étude récente a montré que l'expression constitutive d'un mutant dominant-négatif de RARα dans les cellules suprabasales de l'épiderme d'une souris résulte en une fonction barrière de la peau diminuée (Imakado, S., Bickenbach, J.R., Bundman, D.S., Rothnagel, J.A., Attar, P.S., Wang, X.-J., Walczak, V.R., Wisniewski, S., Pote, J., Gordon, J.S., Heyman, R.A., Evans, R.M. and Roop, D.R. Targeting expression of a dominant-negative retinoic acid receptor mutant in the epidermis of transgenic mice results in loss of barrier function. Genes Dev., 1995, 9, 317-329). Il a été suggéré que le RARα muté soit capable de séquestrer le RXR qui est nécessaire pour la formation de l'hétérodimère PPAR-RXR (Keller, H., Dreyer, C., Medin, J., Mahfoudi, A., Ozato, K. and Wahli, W. Fatty acids and retinoids control lipid metabolism through activation of peroxisome proliferator-activated receptor - retinoid X receptor heterodimers. Proc. Natl. Acad. Sci. USA, 1993; 90; 2160-2164 - Issemann, I., Prince, R.A. Tugwood, J.D. and Green, S. The retinoid X receptor enhances the function of the peroxisome proliferator activated receptor. Biochimie, 1993, 75, 251-256). Ceci implique que l'inactivation de PPARα est en partie responsable de la perte de la fonction barrière de l'épiderme dans les souris transgéniques.

Ainsi, nous démontrons ici que les trois sous-types de PPAR sont exprimés dans les kératinocytes humains. PPARδ montre le niveau le plus élevé d'expression, tandis que PPARα et PPARγ sont exprimés à un faible niveau. L'absence d'expression du gêne de PPAR, rapporté dans les kératinocytes interfolliculaires du rat (Braissant, O., Foufelle, F., Scotto, C., Dauca, M. and Wahli, W. Differential expression of proxisome proliferator-activated receptor (PPARs) : tissue distribution of PPAR-α, -β, and -γ in the adult rat, Endocrinology, 1996, 137, 354-366), peut être due à la sensibilité insuffisante de la technique d'hybridation et/ou de la différence des espèces étudiées.

Nos résultats montrent clairement que l'expression de PPARγ et, dans une certaine mesure, celle de PPARα est liée à la différenciation des kératinocytes. L'expression de PPARγ dans les KHN cultivés pendant quatre jours dans un milieu haut calcium augmente sensiblement.

Cette observation a été confirmée en utilisant un modèle de peau reconstruite, où l'expression de PPARγ augmente durant la stratification et la kératinisation de l'épiderme. L'expression de PPAR a été comparée à l'augmentation des différents marqueurs (keratin 1, TGI et loricine), normalement exprimés dans le compartiment suprabasal de l'épiderme normal. Ceci suggère fortement que l'expression de PPAR -γ a lieu dans les couches suprabasales de l'épiderme humain et est liée à la différenciation des KHN.

L'expression des sous-types de PPAR a également été déterminée dans des épidermes psoriatiques lésés et non-lésés. Une diminution des niveaux de PPARα et de PPARγ a été observée dans l'épiderme lésé hyperproliférateur, montrant, une fois encore, que l'expression des deux sous-types est dépendante de l'état de différenciation de l'épiderme. L'expression de PPARδ augmente dans des épidermes lésés, ce qui laisse suggérer que l'expression de ce sous-type pourrait être associée à la prolifération des kératinocytes.

En conclusion, nous avons démontré qu'en utilisant des cultures submergées ou exposées à l'air, que l'expression des gènes de PPARα et -γ augmentent pendant la différenciation des kératinocytes, tandis que l'expression de PPARδ n'est pas modifiée. Dans l'épiderme psoriasique hyperprolifératif, l'expression de PPAR-γ diminue, ce qui montre que ce sous-type est lié à la différenciation des kératinocytes.

## Revendications

1. Utilisation d'au moins un activateur des récepteurs de type PPAR-γ choisi parmi l'acide-3-{4-[2-(Benzooxazol-2-yl-méthyl-amino)-éthoxy]-phényl}-2-éthoxy-propionique ; l'acide(+)-3-{4-[2-(Benzooxazol-2-yl-méthyl-amino)-éthoxy]-phényl}-2-éthoxy-propionique; et l'acide(-)-3-{4-[2-(Benzooxazol-2-yl-méthyl-amino)-éthoxy]-phényl}-2-éthoxy-propionique dans la préparation d'une composition pharmaceutique, plus particulièrement dermatologique, la composition étant destinée à traiter les désordres cutanés liés à une anomalie de la différenciation des cellules épidermiques choisis parmi le psoriasis, l'eczéma, le lichen plan, les lésions de la peau associées à un lupus, les dermatites, telles que les dermatites atopique, séborrhéique ou solaire, les kératoses, telles que la kératose séborrhéique, sénile, actinique, photo-induite ou folliculaire, l'acné vulgaire, les kéloïdes, les nevi, les verrues, les ichtyoses.

2. Utilisation selon la revendication précédente, **caractérisée en ce que** l'activateur des récepteurs de type PPAR-γ présente une AC50 relative au PPAR-γ inférieure ou égale à 200 nM et avantageusement inférieure ou égale à 50 nM.

3. Utilisation selon la revendication précédente, **caractérisée en ce que** l'activateur des récepteurs de type PPAR-γ est spécifique.

4. Utilisation selon la revendication précédente, **caractérisée en ce que** l'activateur des récepteurs de type PPAR-γ présente un rapport R1 d'AC50 relative au PPAR-γ sur l'AC50 relative au PPARα inférieur ou égal à 0,05, avantageusement inférieur ou égale à 0,02.

5. Utilisation selon l'une des revendications précédentes, **caractérisée en ce que** l'activateur des récepteurs de type PPAR-γ utilisé présente un % d'inhibition de prolifération des kératinocytes inférieur ou égal à 20% lorsqu'il est utilisé à une concentration inférieure ou égale à 100nM.

6. Utilisation selon l'une quelconque des revendications précédentes, **caractérisée en ce que** la composition pharmaceutique est conditionnée sous une forme convenant à une application par voie topique.

7. Utilisation selon la revendication précédente, **caractérisée en ce que** l'activateur des récepteurs de type PPAR-γ est utilisé à une concentration généralement comprise entre 0,001 % et 10 % en poids, de préférence entre 0,01 et 1 % en poids, par rapport au poids total de la composition.

8. Utilisation de la 5-{4-[2-(methyl-pyridin-2-yl-amino)-éthoxy]-benzyl}-thiazolidine-2,4-dione dans la préparation d'une composition pharmaceutique topique, plus particulièrement dermatologique, la composition étant destinée à traiter les désordres cutanés liés à une anomalie de la différenciation des cellules épidermiques choisis parmi le psoriasis, l'eczéma, le lichen plan, les lésions de la peau associées à un lupus, les dermatites, telles que les dermatites atopique, séborrhéique ou solaire, les kératoses, telles que la kératose séborrhéique, sénile, actinique, photo-induite ou folliculaire, l'acné vulgaire, les kéloïdes, les nevi, les verrues, les ichtyoses,
ladite 5-{4-[2-(méthyl-pyridin-2-yl-amino)-éthoxy]-benzyl}-thiazolidine-2,4-dione étant utilisée à une concentration généralement comprise entre 0,001 % et 10 % en poids, de préférence entre 0,01 et 1 % en poids, par rapport au poids total de la composition.

## Claims

1. Use of at least one activator of receptors of PPAR-γ type chosen from 3-{4-[2-(benzoxazol-2-ylmethylamino)ethoxy]phenyl}-2-ethoxypropionic acid; (+)-3-{4-[2-(benzoxazol-2-ylmethylamino)ethoxy]phenyl}-2-ethoxypropionic acid; and (-)-3-{4-[2-(benzoxazol-2-ylmethylamino)ethoxy]phenyl}-2-ethoxypropionic acid for preparing a pharmaceutical, more particularly dermatological, composition, the composition being intended for treating skin disorders related to an abnormality of the differentiation of epidermal cells chosen from psoriasis, eczema, lichen planus, skin lesions associated with lupus, dermatitides such as atopic, seborrho or solar dermatitis, keratoses such as seborrho, senile, actinic, light-induced or follicular keratosis, common acne, keloids, nevi, warts and ichthyoses.

2. Use according to the preceding claim, **characterized in that** the activator of receptors of PPAR-γ type has an AC50 relative to PPAR-γ which is lower than or equal to 200 nM, and advantageously which is lower than or equal to 50 nM.

3. Use according to the preceding claim, **characterized in that** the activator of receptors of PPAR-γ type is specific.

4. Use according to the preceding claim, **characterized in that** the activator of receptors of PPAR-γ type has a ratio R1 of AC50 relative to PPAR-γ over AC50 relative to PPARα which is lower than or equal to 0.05, advantageously lower than or equal to 0.02.

5. Use according to one of the preceding claims, **characterized in that** the activator of receptors of PPAR-γ type used has a percentage of inhibition of keratinocyte proliferation which is less than or equal to 20% when it is used at a concentration which is lower than or equal to 100 nM.

6. Use according to any one of the preceding claims, **characterized in that** the pharmaceutical composition is packaged in a form which is suitable for application via the topical route.

7. Use according to the preceding claim, **characterized in that** the activator of receptors of PPAR-γ type is used at a concentration generally between 0.001% and 10% by weight, preferably between 0.01 and 1% by weight, relative to the total weight of the composition.

8. Use of 5-{4-[2-(methylpyridin-2-ylamino)ethoxy]-benzyl} thiazolidine-2,4-dione for preparing a topical pharmaceutical, more particularly dermatological, composition, the composition being intended for treating skin disorders related to an abnormality of the differentiation of epidermal cells chosen from psoriasis, eczema, lichen planus, skin lesions associated with lupus, dermatitides such as atopic, seborrho or solar dermatitis, keratoses such as seborrho, senile, actinic, light-induced or follicular keratosis, common acne, keloids, nevi, warts and ichthyoses, said 5-{4-[2-(methylpyridin-2-ylamino)ethoxy]benzyl}thiazolidine-2,4-dione being used at a concentration generally between 0.001% and 10% by weight, preferably between 0.01 and 1% by weight, relative to the total weight of the composition.

## Patentansprüche

1. Verwendung mindestens eines Aktivators der Rezeptoren vom Typ PPAR-γ, der unter 3-{4-[2-(Benzooxazol-2-yl-methyl-amino)-ethoxy]-phenyl}-2-ethoxypropionsäure; (+)-3-{4-[2-Benzooxazol-2-yl-methyl-amino)-ethoxy]-phenyl}-2-ethoxypropionsäure; und (-)-3-{4-[2-Benzooxazol-2-yl-methyl-amino)-ethoxy]-phenyl}-2-ethoxypropionsäure ausgewählt ist, für die Herstellung einer pharmazeutischen Zusammensetzung und insbesondere einer dermatologischen Zusammensetzung, wobei die Zusammensetzung dazu vorgesehen ist, Hautstörungen zu behandeln, die mit einer anomalen Differenzierung der Epidermiszellen zusammenhängen und die unter Psoriasis, Ekzemen, Lichen planus, Läsionen der Haut, die mit Lupus einhergehen, Dermatitis, wie atopischer Dermatitis, seborrhoischer Dermatitis oder Dermatitis solaris, Keratosen, wie seborrhoischer Keratose, seniler Keratose, aktinischer Keratose, lichtinduzierter Keratose oder follikulärer Keratose, Akne vulgaris, Keloiden, Naevi, Verruceae und Ichtiosen ausgewählt sind.

2. Verwendung nach dem vorhergehenden Anspruch, **dadurch gekennzeichnet, dass** der Aktivator der Rezeptoren vom Typ PPAR-γ einen auf PPAR-γ bezogenen AC50-Wert kleiner oder gleich 200 nM und vorteilhaft kleiner oder gleich 50 nM aufweist.

3. Verwendung nach dem vorhergehenden Anspruch, **dadurch gekennzeichnet, dass** der Aktivator der Rezeptoren vom Typ PPAR-γ spezifisch ist.

4. Verwendung nach dem vorhergehenden Anspruch, **dadurch gekennzeichnet, dass** der Aktivator der Rezeptoren vom Typ PPAR-γ ein Verhältnis R1 des auf PPAR-γ bezogenen AC50-Wertes und des auf PPAR-α bezogenen AC50-Wertes von kleiner oder gleich 0,05 und vorteilhaft kleiner oder gleich 0,02 aufweist.

5. Verwendung nach einem der vorhergehenden Ansprüche, **dadurch gekennzeichnet, dass** der verwendete Aktivator der Rezeptoren vom Typ PPAR-γ eine prozentuale Inhibierung der Proliferation von Keratinocyten kleiner oder gleich 20 % zeigt, wenn er in einer Konzentration von kleiner oder gleich 100 nM verwendet wird.

6. Verwendung nach einem der vorhergehenden Ansprüche, **dadurch gekennzeichnet, dass** die pharmazeutische Zusammensetzung in einer Form konfektioniert ist, die für eine Anwendung auf topischem Weg geeignet ist.

7. Verwendung nach dem vorhergehenden Anspruch, **dadurch gekennzeichnet, dass** der Aktivator der Rezeptoren vom Typ PPAR-γ in einer Konzentration im Allgemeinen im Bereich von 0,001 bis 10 Gew.-% und vorzugsweise 0,01 bis 1 Gew.-%, bezogen auf das Gesamtgewicht der Zusammensetzung, verwendet wird.

8. Verwendung des 5-{4-[2-(Methylpyridin-2-yl-amino)-ethoxy]-benzyl}-thiazolidin-2,4-dions bei der Herstellung einer topischen pharmazeutischen Zusammensetzung und insbesondere einer dermatologischen Zusammensetzung, wobei die Zusammensetzung dazu vorgesehen ist, Hautstörungen zu behandeln, die mit einer anomalen Differenzierung der Epidermiszellen zusammenhängen und die unter Psoriasis, Ekzemen, Lichen planus, Läsionen der Haut, die mit Lupus einhergehen, Dermatitis, wie atopischer Dermatitis, seborrhoischer Dermatitis oder Dermatitis solaris, Keratosen, wie seborrhoischer Keratose, seniler Keratose, aktinischer Keratose, lichtinduzierter Keratose oder follikulärer Keratose, Akne vulgaris, Keloiden, Naevi, Verruceae und Ichtiosen ausgewählt sind, wobei das 5-{4-[2-(Methylpyridin-2-yl-amino)-ethoxy]-benzyl}-thiazolidin-2,4-dion in einer Konzentration im Allgemeinen im Bereich von 0,001 bis 10 Gew.-% und vorzugsweise 0,01 bis 1 Gew.-%, bezogen auf das Gesamtgewicht der Zusammensetzung, verwendet wird.
